Europäisches Patentamt

**European Patent Office**

**Office européen des brevets**

(11) Publication number: **0 330 313**
**A1**

## EUROPEAN PATENT APPLICATION

(21) Application number: **89300879.7**

(22) Date of filing: **30.01.89**

(51) Int. Cl.⁴: **A61F 5/01**

(30) Priority: **02.02.88 GB 8802290**

(43) Date of publication of application:
**30.08.89 Bulletin 89/35**

(84) Designated Contracting States:
**DE ES FR GB IT SE**

(71) Applicant: **PROTECTAIR LIMITED**
**18-20 Blacklands Way Abingdon Business Park**
**Abingdon Oxon OX14 1DY(GB)**

(72) Inventor: **Young, David Ernest**
**Bowler's Piece 16 Couching Street**
**Watlington Oxon OX9 5QQ(GB)**
Inventor: **Davis, Kenneth Paul**
**7 Dean Close**
**Hillingdon Middlesex, UB10 9UB(GB)**

(74) Representative: **West, Alan Harry et al**
**R.G.C. Jenkins & Co. 26 Caxton Street**
**London SW1H 0RJ(GB)**

(54) **Knee brace.**

(57) A knee brace (1) comprises lateral and medial hinges (3;2) each with proximal and distal hinge arms (6,4; 7,5;) and a transverse connecting bar (10, 21) between the hinges, preferably posteriorly of the knee. The lateral hinge is preferably offset from the lateral aspect of the knee and the medial hinge is preferably closely apposed to the medial aspect of the knee by suitable shaping of the respective proximal and distal hinge arms. The force of a lateral blow to the knee is transmitted through the lateral hinge arms and the transverse connecting bar to divert and attenuate the force of the blow and lessen the risk of injury to the medial co-lateral ligament of the knee.

FIG.1.

## KNEE BRACE

This invention relates to knee braces and more especially to improved sports braces also known as functional braces.

Knee braces are currently widely used in the United States and their use is increasing in other countries, especially in the western hemisphere.

There are several different kinds of knee braces according to the intended application. Thus, minor injuries are often treated with a sewn or wrap-around circumferential sleeve of neoprene, a dense closed-cell synthetic rubber foam which provides compression and retains heat around the affected joint.

In contrast, severe injuries to the cruciate and collateral ligaments of the knee, such as complete rupture, are treated by reconstruction, repair or prosthetic substitution, usually by arthroscopic surgery. It is normal to nurse the knee joint post-operatively for up to eight weeks or even more in a long lever brace with some form of motion control at the knee.

Moderate injuries to the knee ligaments which do not require surgery and knees which have been subjected to surgery but in which a more vigorous regimen is desired are very frequently treated with so-called functional braces. Such braces are intended to allow the knee to move more or less naturally during rehabilitation, including during a return to sporting activities. Some doctors prescribe their use more or less permanently, at least during exercise. Braces of this type are thus deployed as prophylactic devices against further damage to ligaments.

It is to this last type of device that the present invention relates although the invention has some additional relevance to post-operative braces and neoprene knee sleeve braces.

Modern functional knee braces undoubtedly owe their general origins to the Lenox Hill Knee Brace produced at the Lenox Hill Hospital in New York in the 1960s. This brace consists of lateral and medial hinge elements each with two short lever arms. In this context, the term "short lever" simply means short in comparison with arms typically found in post-operative rehabilitation braces. A series of straps and bands is used to secure the brace onto the leg and is alleged to apply various forces about and around the knee. Great emphasis is laid by the manufacturers on the need to custom construct the brace for each patient.

As the techniques for carrying out anterior cruciate ligament repairs improved and demand for the procedure grew, other braces were introduced. Several of these did not espouse the doctrine of customisation, relying instead on a wide variety of sizes. These included the Lerman Knee Brace and the Feanny Knee Orthosis. The hinge of the former is an adaptation of the Lerman cast bracing hinge which has a form of motion control system. The latter features a type of hinge which is very heavy. Both of these braces feature semi-rigid bands which pass half-way around the leg, the remaining hemi-circumferences being taken up by flexible bands which secure the brace on the leg.

In the early 1980s Donjoy Corp of Carlsbad, USA, introduced a short lever sports or functional brace which was lighter than most others in use at that time. However, it used thin plastics for the half bands and loop and pile straps to secure it on the limb.

Over the following few years, a large number of functional or sports braces was introduced, almost all of which employed the same basic theme of medial and lateral hinges each with a proximal and a distal hinge arm, anterior or posterior distal and proximal semi-rigid bands and the whole usually being secured by loop and pile straps. Some of these enjoy considerable popularity in the United States including the C.Ti. brace, Townsend, CanAm and Donjoy 4-point ACL.

A study by Romash et al presented at the 1987 meeting of the American Academy of Orthopaedic Surgeons in San Francisco, compared five braces of the custom type built especially for each patient, with five braces of the off-the-shelf variety. No differences in function were found but the off-the-shelf braces were cheaper. This study was welcomed as it debunked the concept that a custom brace, produced from a cast on an athlete's leg at an early phase of rehabilitation will fit well during later phases when active rehabilitation is taking place. Clearly, in a well muscled athletic leg, wastage will occur in the early weeks after surgery when movement and work are at least partly restricted. Later, bulk will tend to increase which will cause the fit and function of a custom brace to deteriorate if it was indeed produced to "protect" the knee during active rehabilitation to sport.

In the autumn of 1987, Paulos reported that by using a surrogate limb apparatus he had been able to demonstrate a standard to which functional braces should perform in order to protect the medial collateral ligament in the event of the knee receiving a lateral blow. On an index ranging from 1 to 2, a score of 1.8 is the minimum which will provide adequate protection. None of the braces tested achieved that score, the nearest being the Donjoy 4-point ACL.

It is the belief of the present inventors that the design of the known braces are fundamentally flawed in respect of providing protection to the medial collateral ligament, and the present invention seeks to overcome that disadvantage.

More especially, the present invention seeks to provide for a functional or sports brace a transverse coupling element which extends from a lateral hinge member to a medial hinge member and which will in the event of a lateral blow, transmit load from the lateral to the medial side of the brace, minimising the risk of impingement of the lateral hinge against the lateral aspect of the knee and in the event of such impingement, significantly attenuating the amount of energy delivered to the lateral aspect of the knee and ultimately to the medial collateral ligament.

In accordance with the invention, therefore, there is provided a knee brace comprising medial and lateral hinge members each having proximal and distal hinge arms and adapted for fastening to the leg of a patient, and a transverse coupling element which can be secured to the hinge members to extend between them and maintain their transverse spacing at the sides of the patient's knee.

The functional or sports brace of the invention has medial and lateral hinge members each with proximal and distal hinge arms. The hinge members may be unipivotal or bipivotal but are preferably bipivotal, and the body components of the hinge members are suitably cast or machined metal or injection moulded plastics materials.

The two distal hinge arms are suitably united, preferably anteriorly, by a light-weight semi-rigid and generally arcuate band, for example of aluminium alloy. The two proximal arms are preferably similarly united anteriorly and the brace is then able to be secured upon the wearer's leg with a series of posterior loop and pile straps mounted on the proximal and distal arms.

In accordance with the invention, the lateral and medial hinge members are connected together at a desired spacing by a rigid load-transmitting transverse coupling element. Although the transverse element may be circumferential (and, indeed there is no functional reason why this should not be so) when the brace is to be worn for the playing of contact sports, the presence of an anterior bar, even when encased in shock attenuating material, may be regarded as presenting a danger to other players. For this reason, posteriorly mounted hemicircumferential or half-halo load transmitting transverse coupling elements, or elements of other shapes providing adequate load transmitting properties are preferred, especially since it has been possible to devise half-halo transverse coupling elements with no significant reduction in performance over full-halo elements. Nevertheless, it will be appreciated that when the patient is engaged in non-contact sports (for example racquet sports) or in non-contact sports' training or other forms of physical exercise, a circumferential or anterior transverse coupling member may be acceptable or even preferred.

Under most circumstances, however, a posterior transverse element is preferred and will generally be encased in shock attenuating material, at least during sporting use.

According to one embodiment of the invention, the transverse coupling element is constituted by a medial bar which is attached to the posterior edge of the main medial hinge preferably by a suitable hinge means, conveniently a yoke hinge, and a lateral bar which is attached to the posterior edge of the main lateral hinge suitably by means of a positive lock. The medial bar extends posteriorly for a short distance so that it just clears the popliteal surface of the knee and then curves laterally, ending about the mid-point of the popliteal fossa in a male threaded portion. The lateral bar is similarly shaped and extends towards the medial bar, terminating in a male threaded portion of opposite hand to the corresponding portion of the medial bar. An adjuster with female threads of opposite hand at its ends is fitted to the corresponding ends of the medial and lateral bar, so that the transverse length of the bar can be readily adjusted to suit the particular patient.

According to a second embodiment of the present invention, the transverse bar is readily detachable from the hinge elements. This has the advantages that the transverse bar can be removed from the brace when the patient is not engaged in physical activity requiring its presence, and that the patient is then more easily able to wear conventional clothing over the brace. In a preferred arrangement according to the second embodiment, the bar is of one-piece construction and is notched adjacent its ends, the notches being engagable by latches which are themselves detachably secured to the lateral and medial hinges. This may be achieved by concealing the latches with appropriate housings which can in turn be secured to the bodies of the lateral and medial hinges. In this manner, the latches can be arranged to be readily releasable to permit removal of the transverse bar, yet be concealed to prevent their accidental release during sporting activity.

With the prior art braces described above worn during contact sports, when a blow is received to the lateral aspect of the knee, the lateral hinge or parts of the brace arms lying close to it, are driven in against the knee. Thereafter, most of the force of the blow is transmitted directly across the joint, delivering a considerable amount of energy into it. If the foot remains on the ground and the recipient is unable to accelerate a substantial part of his mass in the direction of the blow, inertia is likely to be such that the lateral blow may succeed in deforming the knee medially to the extent that the ligamentous structure is ruptured. Obviously the medial collateral ligaments is at greater risk but in the case of an undefended blow from a 130 kg man travelling horizontally at about 12 m/s, the cruciate ligaments, whether repaired or not, are obviously at risk, too. This type of blow is called a "clipping" injury in American gridiron football, but the resulting injury is common in soccer, rugby football and other contact sports, also.

In this type of incident, deformation of the brace has had no significant part to play in defending the medial collateral ligament. In general, modern brace designs have the lateral hinge and arms closely apposed to the lateral aspect of the knee, to the extent that in many cases they are virtually close coupled. Because of this, even if the distal and proximal half-bands were so configured and of such a material as to attenuate the load in the very small time transient that it is delivered, they never get the chance so to respond.

In the brace according to the present invention however, the provision of a transverse load transmitting element between the lateral hinge member · which is preferably offset from the lateral aspect of the knee, and the medial hinge member which may if so desired be apposed to the medial aspect of the knee, effectively provides decoupling of the brace from the knee at the point of delivery of the most dangerous blows, so far as the medial collateral ligament is concerned. The load transmitting element and the brace hinge/arm members then act as a load attenuating structure by provision of two single-element leaf springs coupled in the centre and substantially anchored at the ends. Anterior half-bands which fit around the limb proximally and distally also contribute, though minimally, to attenuating a lateral blow provided that the stiffness of the brace hinge/arm members exceeds the inherent stiffness of the half-bands.

The soft tissues of the lateral aspect of the limb, proximal and distal to the knee and under that portion of the brace where the brace hinge/arm and anterior half bands are jointed will also play a small part in the process of attenuating the force of the blow.

The invention will now be described in greater detail by way of example only with reference to the accompanying drawings in which

Fig. 1 is a perspective view of a first embodiment of a sports brace according to the invention, showing the lateral and medial hinges and parts of their respective hinge arms together with a transverse load-transmitting coupling element;

Fig. 2 is a plan view of the coupling element shown in Fig. 1;

Fig. 3 is a perspective view of part of a sports brace according to a second embodiment of the invention, showing the lateral hinge and parts of its hinge arms and the lateral end of the coupling element;

Fig. 4 is a side view of the lateral hinge shown in Fig. 3, showing in hidden detail the attachment of the coupling element to the hinge; and

Fig. 5 is a plan view of a sports brace according to the second embodiment but with retaining straps omitted.

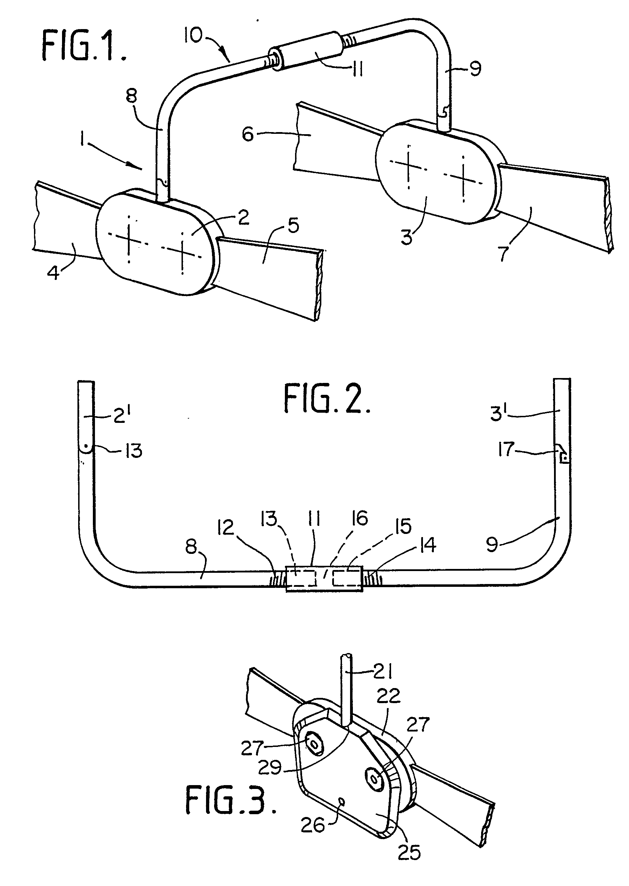

Referring first to Fig. 1 of the drawings, a sports brace 1 has a lateral hinge 2 and a medial hinge 3 with proximal hinge arms 4 and 6 and distal hinge arms 5 and 7, respectively. A transverse coupling element 10 connects the two hinges 2 and 3 and consists of a lateral bar 8 and medial bar 9 joined together by an adjuster 11.

Turning now to Fig. 2, it will be seen that lateral bar 8, has a free end 2′ by means of which it is attached to lateral hinge 2, a yoke hinge 13 and a male threaded portion 12 at its other end. Male threaded portion 12 is engaged by the female threaded portion 13 of adjuster 11. Medial bar element 9 has a male threaded portion 14, which is engaged by the female threaded portion 15 of adjuster 11. Male threaded portion 14 has the opposite handed thread to threaded portion 12; it follows that the female threaded portions 13 and 15 of adjuster 11 also have corresponding opposite handed threads. The female threaded portions 13 and 15 are separated by a central solid segment 16 which serves to maintain an equal extent of adjustment on lateral bar 8 and medial bar 9. Medial bar 9 is attached to medial hinge 3 at its free end 3′ via a latch lock 17.

The hinges 2 and 3 are shown in Fig. 1 in outline only since their precise nature is of little importance to the transverse coupling element which extends between them. However, it will be noted from Fig. 1 that the hinges 2 and 3 are represented generally as bipivotal hinges, that is to say hinges in which the medial and proximal hinge arms have separate pivots spaced a small distance apart. Such bipivotal hinges have been demonstrated to follow more closely the flexion and extension movement of the knee, than unipivotal hinges.

In the knee brace shown in Fig. 1, the lateral and medial bars 8 and 9 are shown as being inserted directly into the bodies of hinges 2 and 3, respectively. The hinge bodies will generally be metal casting or plastics injection mouldings and may therefore be formed around the ends of the bars 8 and 9, if desired. Alternatively, if permanent attachment of the bars to the hinge bodies is not required, then the bars can be detachably accommodated in mating recesses in the hinge bodies, for example as a push fit, if necessary with a grub screw or similar fastening, as a snap fit or with complementary threading.

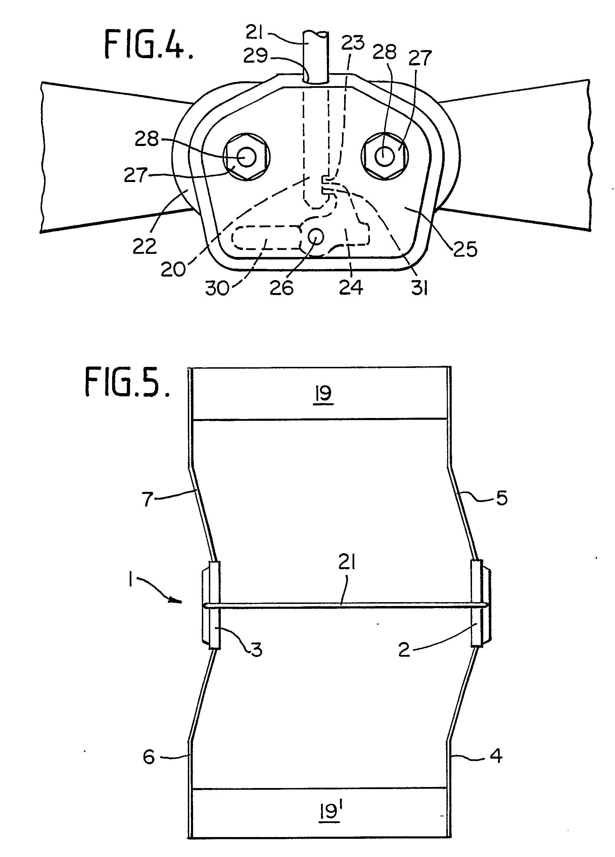

Figs. 3 and 4 show an alternative and preferred means of attaching the transverse coupling element to the hinge members and illustrates only attachment of lateral end 20 of the coupling element 21 to the lateral hinge 22, it being understood that the medial end of the coupling element 21 will be attached to the medial hinge in an identical manner. In this preferred embodiment, the coupling element 21 is provided adjacent its lateral end 20 with a notch 23 which is engaged by a spring-biased latch 24, as shown in hidden detail in Fig. 4. The latch 24 is accommodated within a housing 25, for example of injection moulded plastics material, and pivots about a pin 26 fastened to the housing 25 and is biased by a spring (not shown) into the position shown in Fig. 4 in which latching finger 31 engages the notch 23. The housing 25 is fastened to the hinge 2 by means of nuts 27 which serve to retain the pivot pins 28 of the hinge.

In order to fasten the coupling element 21 to the hinge 2 to which the housing 25 and its latch 24 have already been attached, the free end of the coupling element is inserted through the aperture 29 in the housing and pushed forward until the notch 23 is engaged by the finger 31. To disengage the coupling element, the latch is released by applying finger pressure to a tongue 30 which projects from the latch 24 below the edge of the housing 25.

Fig. 5 shows the complete sports brace 1, comprising lateral hinge 2 and medial hinge 3 and their respective proximal hinge arms 4 and 6 and distal hinge arms 5 and 7, with transverse coupling element 21 latched in place, together with distal anterior half-band 19 and proximal anterior half-band 19'. Retaining straps which are necessary for fastening the brace to the patient's leg are omitted for clarity.

As can be seen in Fig. 5 hinge arms 5 and 7 are offset laterally to provide clearance between the lateral aspect of the knee and the inner aspect of lateral hinge 2. Hinge arms 6 and 7 are inset in this preferred embodiment, although it is possible for hinge arms 6 and 7 not to be offset or for hinge arm 6 to be offset and hinge arm 7 not be offset, and vice versa.

Adjuster 11 shown in Figs. 1 and 2 is used to get the best "lie" of the brace on the patient's leg. It is possible to construct a brace in which proximal arms are made from different material to that used for distal arms and also in which one pair of diagonally opposite arms are made of the same material which differs from the material from which the second diagonally opposite pair of arms is made.

It is also possible to manufacture sports braces according to the invention in which the lateral and medial bar elements do not have threaded ends and the adjuster is a self contained sub-assembly mounted between them; in which a single bar without an adjuster extends between the lateral and medial hinges; and having an entire anterior bar and interrupted posterior bar. Padded and unpadded versions have been made.

Steel, titanium, aluminium alloys and plastics have all been employed as materials for hinge arms and for elements of the load transmitting coupling element.

It is possible to make use of the invention in long lever post-operative rehabilitative knee braces as a prophylactic against trauma and in neoprene knee sleeves fitted with knee hinges again as a prophylactic against trauma.

## Claims

1. A knee (1) brace comprising medial and lateral hinge members (3;2) each having proximal and distal hinge arms (6,4; 7,5) and adapted for fastening to the leg of a patient, and a transverse coupling element (10, 21) which can be secured to the hinge members to extend between them and maintain their transverse spacing at the sides of the patient's knee.

2. A knee brace according to claim 1, wherein the transverse coupling element (10, 21) can be secured to the hinge members to extend between them anteriorly.

3. A knee brace according to claim 1, wherein the transverse coupling element (10, 21) can be secured to the hinge members to extend between them posteriorly.

4. A knee brace according to any one of claims 1 to 3, wherein the length of the transverse coupling element (10) is adjustable (11) to suit the desired spacing between the hinge members.

5. A knee brace according to any one of claims 1 to 4, wherein the transverse coupling element (21) is readily detachable from the hinge members.

6. A knee brace according to claim 5, wherein the transverse coupling element (21) is securable to the hinge members at its ends (20) via releasable latches (24) which are themselves detachably secured to the hinge members.

7. A knee brace according to any one of claims 1 to 6, wherein the hinge members are bipivotal hinges.

8. A knee brace according to any one of claims 1 to 7, wherein the proximal and distal arms (4;5) of the lateral hinge member (2) are offset to space the inner aspect of the lateral hinge member from the lateral aspect of the patient's knee.

9. A knee brace according to any one of claims 1 to 8, wherein the proximal and distal arms (6;7) of the medial hinge member (3) are so shaped that the inner aspect of the medial hinge member is closely apposed to the medial aspect of the knee.

10. A knee brace according to any one of claims 1 to 9, wherein the medial and lateral proximal hinge arms (6;4) and the medial and lateral distal hinge arms (7;5), respectively, are connected together by hemi-circumferential bands (19';19).

Neu eingereicht / Newly filed
Nouvellement déposé

FIG.1.

FIG.2.

FIG.3.

3d eingereicht / Newly filed
Nouvellement déposé

FIG.4.

FIG.5.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | US-A-3 958 569 (VOSBURGH) <br> * figures 1,2; column 1, lines 16-30 * | 1 | A 61 F 5/01 |
| A | | 2,5,7, 10 | |
| X | MEDIZINISCH-ORTHOPAEDISCHE TECHNIK vol. 107, no. 2, 1987, pages 64-67; W. KNOCHE: "Knieorthesen". * figure 21; page 67, left hand column, lines 12-31 * | 1 | |
| A | idem | 3-10 | |
| X | US-A-2 144 641 (SNYDER) <br> * claim 1; figures 1,2 * | 1 | |
| A | | 2,3 | |
| X | WO-A-8 707 828 (CADORET) <br> * claim 1; figures 1,2 * | 1 | |
| A | | 2,7 | TECHNICAL FIELDS SEARCHED (Int. Cl.4) |
| A | US-A-2 587 166 (JOVICK) <br> * figure 1; claim 1 * | 1 | A 61 F 5/00 |
| A | GB-A-1 180 903 (NATIONAL RESEARCH DEVELOPMENT CORP.) <br> * figures 1,2; page 2, lines 59-64 * | 1 | |
| A | GB-A- 766 924 (ELLIS, SON & PARAMORE LTD.) <br> * figure 4 * | 1 | |

The present search report has been drawn up for all claims

| Place of search | Date of completion of the search | Examiner |
|---|---|---|
| BERLIN | 10-05-1989 | KANAL P K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)